Europäisches Patentamt

European Patent Office

Office européen des brevets

(11) Veröffentlichungsnummer: **0 166 903 B1**

(12) **EUROPÄISCHE PATENTSCHRIFT**

(45) Veröffentlichungstag der Patentschrift: **06.03.91**

(21) Anmeldenummer: **85105327.2**

(22) Anmeldetag: **02.05.85**

(51) Int. Cl.⁵: **C12P 13/04**, C12P 13/06, C12P 13/08, C12P 13/22, //(C12P13/04,C12R1:13,1:15)

(54) Verfahren zur fermentativen Herstellung von verzweigtkettigen aliphatischen oder aromatischen L-Aminosäuren aus alpha-Ketocarbonsäuren.

(30) Priorität: 25.05.84 DE 3419585
28.09.84 DE 3435674

(43) Veröffentlichungstag der Anmeldung:
08.01.86 Patentblatt 86/02

(45) Bekanntmachung des Hinweises auf die
Patenterteilung:
06.03.91 Patentblatt 91/10

(84) Benannte Vertragsstaaten:
BE CH DE FR GB LI NL SE

(56) Entgegenhaltungen:
EP-A- 0 132 999
EP-A- 0 151 488
FR-A- 2 550 224

CHEMICAL ABSTRACTS, Band 74, Nr. 3, 18. Januar 1971, Seite 103, Zusammenfassung Nr. 10662j, Columbus, Ohio, US; Y. TOKORO et al.: "Microbial production of L-phenylalanine from n-alkanes"

CHEMICAL ABSTRACTS, Band 102, Nr. 17,

April 1985, Seite 457, Zusammenfassung Nr. 147439h, Columbus, Ohio, US; E. BULOT: "Selective production of phenylalanine from phenylpyruvate using growing cells of corynebacterium glutamicum"

(73) Patentinhaber: **Degussa Aktiengesellschaft**
**Weissfrauenstrasse 9**
**W-6000 Frankfurt am Main 1(DE)**

Patentinhaber: **Forschungszentrum Jülich GmbH**
**Postfach 1913**
**W-5170 Jülich(DE)**

(72) Erfinder: **Groeger, Ulrich**
**Wendelinusstrasse 1**
**W-5170 Jülich(DE)**
Erfinder: **Sahm, Hermann, Prof.**
**Wendelinusstrasse 71**
**W-5170 Jülich(DE)**
Erfinder: **Leuchtenberger, Wolfgang, Dr.**
**Geschw.-Scholl-Strasse 1**
**W-6454 Bruchköbel(DE)**

## Beschreibung

Die Erfindung betrifft ein Verfahren zur fermentativen Herstellung von verzweigtkettigen aliphatischen oder aromatischen L-Aminosäuren aus den analogen ∝-Ketocarbonsäuren in einem Kulturmedium.

Als für Mensch und Tier essentielle Aminosäuren haben L-Leucin, L-Isoleucin und L-Valin große Bedeutung. Neben der klassischen Gewinnung im großen Maßstab durch Hydrolyse von Proteinen sind in den letzten zwanzig Jahren insbesondere mikrobielle und enzymatische Syntheseverfahren entwickelt worden. Bei den mikrobiellen Syntheseverfahren finden hauptsächlich Vertreter der Gattungen Corynebacterium, Brevibacterium und Arthrobacter Verwendung, deren Regulationsmechanismen der Biosynthesewege für verzweigtkettige Aminosäuren durch Mutation so verändert wurden, daß die Mutanten große Mengen dieser Aminosäuren aus fermentierbaren Kohlenstoffquellen bilden und im Fermentationsmedium akkumulieren (z.B. Isoleucin mit Brevibacterium thiogenitalis, US-PS 4 329 427). Beispiele für die Kohlenstoffquellen sind Kohlenhydrate wie Glucose, Fructose, Maltose, Stärke-Hydrolysat, Cellulose-Hydrolysate und Melassen, organische Säuren wie Acetat und Succinat, Alkohole wie Glycerin und Ethanol und Kohlenwasserstoffe wie n-Paraffine. Beispiele gängiger Stickstoffquellen beinhalten Ammoniumsulfat, Ammoniumchlorid, Ammoniumnitrat, Harnstoff und Ammoniate. Anorganische Salze wie Phosphate und Sulfate sowie Calcium, Magnesium, Mangan und Eisen finden Verwendung. Zusätze von Vitaminen wie Biotin und Thiamin oder komplexer Nährstoffe wie Hefeextrakt, Pepton, Casamino-Acids und Sojabohnen-Hydrolysate ermöglichen ein optimales Wachstum der Mikroorganismen.

Die mikrobielle Gewinnung von Aminosäuren aus Vorstufen ist für einige Aminosäuren, wie Tryptophan aus Indol und Serin oder Anthranilat, Isoleucin aus L-Aminobutyrat, D-Threonin, L-Hydroxybutyrat oder DL-∝-Bromobutyrat ist bekannt.

Die Gewinnung von Aminosäuren aus ihren analogen ∝-Ketocarbonsäuren konnte für L-Glutamat aus ∝-Ketoglutarat (Otsuka et al, I. Gen. Appl. Microbiol. 3 (1)35-53, 1957) und für DL-Alanin aus Pyruvat (Shah et al, I. Gen. Microbiol. 17 , 620-624, 1957) gezeigt werden. Dagegen verliefen Versuche zur mikrobiellen Gewinnung von L-Leucin aus ∝-Ketoisocaproat und L-Valin aus ∝-Ketoisovalerat mit Bacillus subtilis wenig vielversprechend.

Die eingesetzten Mikroorganismen waren zur Gewinnung der verzweigtkettigen, aliphatischen Aminosäuren aus ihren analogen ∝ -Ketocarbonsäuren ungeeignet, da sie den größten Teil der Ketosäuren decarboxylierten, und somit nur geringe Ausbeuten erzielt werden konnten.

Gegenstand der Erfindung ist ein Verfahren zur fermentativen Herstellung von verzweigtkettigen, aliphatischen oder aromatischen L-Aminosäuren aus den analogen ∝-Ketocarbonsäuren in einem Kulturmedium, der dadurch gekennzeichnet ist, daß man Glutamat-produzierende Bakterien auf die ∝-Ketocarbonsäuren einwirken läßt und nach Beendigung der Umsetzung die gewünschte Aminosäure isoliert.

Geeignet sind Vertreter der Gattung Corynebacterium, insbesondere Corynebacterium glutamicum, speziell Corynebacterium glutamicum ATCC 13032, und Vertreter der Gattung Brevibacterium, insbesondere Brevibacterium flavum, speziell Brevibacterium flavum ATCC 14067.

In dem Kulturmedium sind assimilierbare Kohlenstoff- und Stickstoffquellen enthalten. Als Kohlenstoffquelle setzt man bevorzugt eine Kohlehydrat wie z.B. Glucose oder eine niedere Fettsäure bzw. deren Na-Salz wie z.B. Natriumacetat ein. Diese Stoffe haben in dem erfindungsgemäßen Verfahren hauptsächlich eine Bedeutung als Kohlenstoff- und Energiequelle für das Wachstum der Mikroorganismen und als Energiequelle für die Umwandlung der ∝-Ketocarbonsäuren in die entsprechenden L-Aminosäuren. Da die Bildung der Aminosäuren aus den analogen ∝-Ketocarbonsäuren in dem vorliegenden Prozeß nicht an das Wachstum der Bakterien gekoppelt ist, erlaubt dies gleichzeitig, die Konzentration assimilierbarer Kohlenstoffquellen niedrig zu halten und die ∝-Ketocarbonsäure z.B. in einem Fermenter mit Biomasse-Rückhaltung kontinuierlich in die analoge Aminosäure umzuwandeln und hohe Produktkonzentrationen zu erzielen, wobei die Bildung von Nebenprodukten weitgehend unterdrückt wird. Demgegenüber laufen übliche Biosynthesen zur Herstellung z.B. von Isoleucin aus fermentierbaren Kohlenstoffquellen batchweise ab und führen nur zu geringen und so die Aufarbeitung erschwerenden Aminosäurekonzentrationen.

Als ∝-Ketocarbonsäuren besonders geeignet sind ∝-Ketoisocapronsäure, ∝-Ketoisovaleriansäure, ∝-Keto-$\beta$-methylvaleriansäure und Phenylbrenztraubensäure.

Die Fermentation wird vorzugsweise bei einem pH-Wert von 5 bis 9 bei einer Temperatur von 25 bis 40° C unter aeroben Bedingungen für 2 bis 10 Tage durchgeführt. Das Nährmedium weist insb. eine Biotin-Konzentration von mehr als 50 μg/l auf. Bei niedrigeren Biotin-Konzentrationen ist das Wachstum suboptimal. Dies kann eine Erniedrigung der Ausbeute zur Folge haben. Die Ausgangskonzentration des Fermentationsmediums an ∝-Ketocarbonsäure sollte 30 g/l nicht übersteigen, da bei höheren Konzentrationen eine signifikante Wachstumshemmung auftritt. Durch kontinuierliches Nachdosieren der Vorstufe lassen sich jedoch unter Umgehung kritischer Ketosäurekonzentrationen hohe Aminosäurekonzentrationen erreichen.

Die Isolierung und Reinigung der Aminosäure aus der Fermentationsbrühe wird durch konventionelle Methoden vorgenommen.

Das gemäß der Erfindung produzierte L-Leucin, L-Isoleucin, L-Valin oder L-Phenylalanin wurde durch einen Aminosäure-Analysator sowie einen enzymatischen Test mit L-Aminosäure-Oxidase identifiziert und quantifiziert.

Beispiel 1:

In einem 500 ml Erlenmeyerkolben mit 2 Schikanen wurden 100 ml einer Nährlösung mit folgender Zusammensetzung gegeben:

| | |
|---|---|
| 40 | g/l Glucose x $H_2O$ |
| 20 | g/l Na-$\alpha$-Ketoisocapronsäure |
| 20 | g/l $(NH_4)_2SO_4$ |
| 0.5 | g/l $K_2HPO_4$ |
| 0.5 | g/l $KH_2PO_4$ |
| 0.25 | g/l $MgSO_4$ x 7 $H_2O$ |
| 0.01 | g/l $FeSO_4$ x 7 $H_2O$ |
| 0.01 | g/l $MnSO_4$ x 4 $H_2O$ |
| 0.4 | mg/l Biotin |
| 2 | mg/l Thiamindichlorid |
| 20 | g/l $CaCO_3$ |

Vor dem Autoklavieren (20 min bei 121° C und 1 atm Druck) wurde der pH-Wert mit 2 N NaOH auf 7.4 eingestellt. Glucose und $\alpha$-Ketoisocaproat wurden getrennt autoklaviert, Thiamindichlorid sterilfiltriert und $CaCO_3$ trocken sterilisiert (8 h bei 150° C) und nach dem Erkalten der Lösungen steril zugegeben.

Eine 15 Stunden alte Vorkultur von Corynebacterium glutamicum ATCC 13032, gewachsen in 100 ml Komplexmedium (20 g/l Glucose, 10 g/l Pepton, 10 g/l Hefeextrakt, 2.5 g/l NaCl, pH 7.4) in einem 500 ml Erlenmeyerkolben mit 2 Schikanen bei 30° C und 100 rpm wurde 2x in steriler 0.9% NaCl gewaschen und in 20 ml steriler 0.9% NaCl resuspendiert. 2 ml dieser Zellsuspension dienten als Inokulum der Hauptkultur, die bei 30° C und 100 rpm inkubiert wurde. Nach einer Kultivierungsdauer von 57 Stunden enthielt das Fermentationsmedium 15.8 g/l L-Leucin. Das Fermentationsmedium des Kontrollansatzes ohne $\alpha$-Ketoisocaproat wies kein L-Leucin auf.

Beispiel 2:

Es wurde wie in Beispiel 1 beschrieben verfahren, jedoch Brevibacterium flavum ATCC 14067 verwendet. Nach einer Kultivierungsdauer von 72 Stunden enthielt das Fermentationsmedium 14.5 g/l L-Leucin.

Beispiel 3:

Es wurde wie in Beispiel 1 beschrieben verfahren, jedoch enthielt die Nährlösung 40 g/l Na-Acetat anstelle von Glucose.
Nach einer Kultivierungsdauer von 72 Stunden enthielt das Fermentationsmedium 10.9 g/l L-Leucin.

Beispiel 4:

Es wurde wie in Beispiel 3 beschrieben verfahren, jedoch Brevibacterium flavum ATCC 14067 verwendet. Nach einer Kultivierungsdauer von 96 Stunden enthielt das Fermentationsmedium 7.1 g/l L-Leucin.

Beispiel 5:

Es wurde wie in Beispiel 1 beschrieben verfahren, jedoch enthielt die Nährlösung 60 g/l Glucose, 30 g/l Na-$\alpha$-Ketoisocapronsäure und 40 g/l $(NH_4)_2SO_4$. Nach einer Kultivierungsdauer von 96 Stunden enthielt das Fermentationsmedium 21.8 g/l L-Leucin.

Beispiel 6:

Da bei einer Ausgangskonzentration von mehr als 3% w/v $\alpha$-Ketoisocaproat eine signifikante Wachstumshemmung auftritt, wurde durch Nachfüttern von $\alpha$-Ketoisocaproat unter Umgehung hoher $\alpha$-Ketoisocaproat-Konzentrationen eine hohe L-Leucin-Konzentration erreicht.
Es wurde wie in Beispiel 1 beschrieben verfahren, jedoch enthielt die Nährlösung 60 g/l Glucose und 40 g/l $(NH_4)_2SO_4$. Nach einer Inkubationszeit von 48 Stunden wurden 2% w/v Na-$\alpha$-Ketoisocapronsäure nachdosiert und für weitere 53 Stunden inkubiert. Bei dieser Verfahrensweise konnten 24.9 g/l L-Leucin im Fermentationsmedium erreicht werden.

Beispiel 7:

Es wurde wie in Beispiel 1 beschrieben verfahren, jedoch enthielt die Nährlösung 20 g/l Na-$\alpha$-Ketoisovaleriansäure. Nach einer Kultivierungsdauer von 48 Stunden enthielt das Fermentationsmedium 12.3 g/l L-Valin.

Beispiel 8:

Es wurde wie in Beispiel 1 beschrieben verfahren, jedoch enthielt die Nährlösung 20 g/l Na-D/L-α-Keto-β-methylvaleriansäure. Nach einer Kultivierungsdauer von 96 Stunden enthielt das Fermentationsmedium 4,8 g/l L-threo-Isoleucin und 1,8 g/l L-allo-Isoleucin.

Beispiel 9:

Es wurde wie in Beispiel 1 beschrieben verfahren, jedoch enthielt die Nährlösung 60 g/l Glucose x $H_2O$, 40 g/l $(NH_4)_2SO_4$ und 20 g/l Ca-α-Ketoisocapronsäure. Da das Ca-Salz der α-Ketoisocapronsäure schwer löslich ist, wurde es in ungelöster Form zusammen mit den anderen Bestandteilen der Nährlösung autoklaviert. Nach einer Kultivierungsdauer von 72 Stunden enthielt das Fermentationsmedium 12,5 g/l L-Leucin. Der Kontrollansatz mit dem Na-Salz der α-Ketoisocapronsäure enthielt 12,3 g/l L-Leucin.

Die vorstehend für verzweigtkettige aliphatische Aminosäuren beschriebene Umsetzung kann auch für die Umwandlung von aromatischen α-Ketosäuren in die entsprechenden aromatischen α-Aminosäuren wie z.B. Phenylalanin angewandt werden.

Beispiel 10:

Es wurde wie in Beispiel 9 beschrieben verfahren, jedoch enthielt die Nährlösung 10 g/l Ca-Phenylpyruvat. Nach einer Kultivierungsdauer von 48 Stunden enthielt das Fermentationsmedium 4.1 g/l L-Phenylalanin.

Beispiel 11:

Es wurde, wie in Beispiel 1 beschrieben, verfahren, jedoch enthielt die Nährlösung 60 g/l Glucose x $H_2O$ und 40 g/l $(NH_4)_2SO_4$. Nach einer Kultivierungsdauer von 48 Stunden wurden die Zellen durch Zentrifugieren von der Nährlösung getrennt, 2 x in steriler 0.9% Nacl-Lösung gewaschen und in 35 ml steriler 0.9% NaCl-Lösung resuspendiert. 2 ml dieser Zellsuspension wurden zu 8 ml einer sterilen Inkubationslösung zugegeben, bestehend aus 5 ml eines 0.2 M Tris-HCl Puffers pH 8.0, 1 ml einer 1M Glucoselösung, 1 ml einer 1M $(NH_4)_2SO_4$ Lösung und 1 ml einer 1M Na-α-Ketoisocaproatlösung. Nach einer Inkubationsdauer von 48 Stunden bei 30° C und 100 rpm enthielt die Inkubationslösung 0.0943 Mol/l L-Leucin, entsprechend 94.3% Ausbeute, bezogen auf die eingesetzte Menge an Na-α-Ketoisocaproat.

**Ansprüche**

1. Verfahren zur fermentativen Herstellung von verzweigtkettigen aliphatischen oder aromatischen L-Aminosäuren aus den analogen α-Ketocarbonsäuren in einem Kulturmedium, dadurch gekennzeichnet, daß man einen Stamm von Corynebacterium glutamicum oder Brevibacterium flavum auf die α-Ketocarbonsäuren einwirken läßt und nach Beendigung der Umsetzung die entstandene Aminosäure isoliert.

2. Verfahren nach Anspruch 1, dadurch gekennzeichnet, daß man bei einem pH-Wert von 5 bis 9, einer Temperatur von 20 bis 45° C unter aeroben Bedingungen für eine Dauer von 1 bis 10 Tagen arbeitet, und daß das Kulturmedium eine assimilierbare Kohlenstoff- und Stickstoffquelle enthält.

3. Verfahren nach den Ansprüchen 1 bis 2, dadurch ge kennzeichnet, daß man wachsende Zellen verwendet.

4. Verfahren nach den Ansprüchen 1 bis 2, dadurch ge kennzeichnet, daß man nicht-wachsende ("ruhende") Zellen verwendet.

5. Verfahren nach den Ansprüchen 1 bis 4, dadurch ge kennzeichnet, daß die Konzentration der α-Ketocarbonsäure 30 g/l nicht überschreitet.

6. Verfahren nach den Ansprüchen 1 bis 5, dadurch ge kennzeichnet, daß die Biotinkonzentration > als 50 μg/l ist.

7. verfahren nach den Ansprüchen 1 bis 6, dadurch ge kennzeichnet, daß die verwendete α-Ketocarbonsäure α-Ketoisocapronsäure, α-Ketoisovaleriansäure oder α-Keto-β-methylvaleriansäure ist.

8. Verfahren nach Anspruch 1, dadurch gekennzeichnet, daß man Corynebacterium glutamicum ATCC 13032 verwendet.

9. Verfahren nach Anspruch 1, dadurch gekennzeichnet, daß man Brevibacterium flavum ATCC 14067 verwendet.

Patentansprüche für die Vertragsstaaten DE. FR. GB

1. Verfahren zur fermentativen Herstellung von verzweigtkettigen aliphatischen L-Aminosäuren aus den analogen α-Ketocarbonsäuren in einem Kulturmedium,
dadurch gekennzeichnet, daß man einen Stamm von Corynebacterium glutamicum oder Brevibacterium flavum auf die ∝ -Ketocarbonsäuren einwirken läßt und nach Beendigung der Umsetzung die entstandene Aminosäure isoliert.

2. Verfahren nach Anspruch 1, dadurch gekennzeichnet, daß man bei einem pH-Wert von 5 bis 9, einer Temperatur von 20 bis 45° C unter aeroben Bedingungen für eine Dauer von 1 bis 10 Tagen arbeitet, und daß das Kulturmedium eine assimilierbare Kohlenstoff- und Stickstoffquelle enthält.

3. Verfahren nach den Ansprüchen 1 bis 2, dadurch ge kennzeichnet, daß man wachsende Zellen verwendet.

4. Verfahren nach den Ansprüchen 1 bis 2, dadurch ge kennzeichnet, daß man nicht-wachsende ("ruhende") Zellen verwendet.

5. Verfahren nach den Ansprüchen 1 bis 4, dadurch ge kennzeichnet, daß die Konzentration der ∝ -Ketocarbonsäure 30 g/l nicht überschreitet.

6. Verfahren nach den Ansprüchen 1 bis 5, dadurch ge kennzeichnet, daß die Biotinkonzentration > als 50 μg/l ist.

7. Verfahren nach den Ansprüchen 1 bis 6, dadurch ge kennzeichnet, daß die verendete ∝ - Ketocarbonsäure ∝ -Ketoisocapronsäure, ∝ - Ketoisovaleriansäure oder ∝ -Keto-$\beta$-methylvaleriansäure ist.

8. Verfahren nach Anspruch 1, dadurch gekennzeichnet, daß man Corynebacterium glutamicum ATCC 13032 verwendet.

9. Verfahren nach Anspruch 1, dadurch qekennzeichnet, daß man Brevibacterium flavum ATCC 1067 verwendet.

**Claims**

1. Process for the fermentative preparation of branched chain aliphatic or aromatic L-amino acids from the analogous ∝-keto carboxylic acids in a culture medium, characterised in that a stock of corynebacterium glutamicum or brevibacterium flavum is caused to act on the ∝-keto carboxylic acids and the amino acid formed is isolated after termination of the reaction.

2. A process according to Claim 1, characterised in that a temperature from 20 to 45° C is employed under aerobic conditions at a pH from 5 to 9 for a duration of 1 to 10 days and in that the culture medium contains a source of assimilable carbon and nitrogen.

3. A process according to Claims 1 to 2, characterised in that growing cells are used.

4. A process according to Claims 1 to 2, characterised in that non-growing ("resting") cells are used.

5. A process according to Claims 1 to 4, characterised in that the concentration of ∝-keto carboxylic acid does not exceed 30 g/1.

6. A process according to Claims 1 to 5, characterised in that the biotin concentration is greater than 50 μg/l.

7. A process according to Claims 1 to 6, characterised in that the ∝-keto carboxylic acid used is ∝-ketoisocaproic acid, ∝-ketoisovaleric acid or ∝-keto-$\beta$-methylvaleric acid.

8. A process according to Claim 1, characterised in that corynebacterium glutamicum ATCC 13032 is used.

9. A process according to Claim 1, characterised in that brevibacterium flavum ATCC 14067 is used.

Claims for the Contracting States : DE, FR, GB

1. Process for the fermentative preparation of branched chain aliphatic L-amino acids from the analogous ∝-keto carboxylic acids in a culture medium, characterised in that a stock of corynebacterium glutamicum or brevibacterium flavum is caused to act on the ∝-keto carboxylic acids and the amino acid formed is isolated after termination of the reaction.

2. A process according to Claim 1, characterised in that a temperature from 20 to 45° C is employed under aerobic conditions at a pH from 5 to 9 for a duration of 1 to 10 days and in that the culture medium contains a source of assimilable carbon and nitrogen.

3. A process according to Claims 1 to 2, characterised in that growing cells are used.

**4.** A process according to Claims 1 to 2, characterised in that non-growing ("resting") cells are used.

**5.** A process according to Claims 1 to 4, characterised in that the concentration of ∝-keto carboxylic acid does not exceed 30 g/l.

**6.** A process according to Claims 1 to 5, characterised in that the biotin concentration is greater than 50 μg/l.

**7.** A process according to Claims 1 to 6, characterised in that the ∝-keto carboxylic acid used is ∝-ketoisocaproic acid, ∝-ketoisovaleric acid or ∝-keto-$\beta$-methylvaleric acid.

**8.** A process according to Claim 1, characterised in that corynebacterium glutamicum ATCC 13032 is used.

**9.** A process according to Claim 1, characterised in that brevibacterium flavum ATCC 14067 is used.

**Revendications**

**1.** Procédé permettant d'obtenir par fermentation des acides aminés ramifiés aliphatiques ou aromatiques à partir de leurs homologues acides ∝ cétoniques, dans un milieu de culture, caractérisé en ce que l'on fait agir une souche de corynebacterium glutumicum ou de brevibacterium flavum sur les acides a cétoniques, et qu'à l'issue de la transformation, on isole les acides aminés formés.

**2.** Procédé conforme à la revendication 1, caractérisé en ce que l'on travaille à un pH compris entre 5 et 9, à une température comprise entre 20 et 45° C, en conditions aérobies pendant une durée de 1 à 10 jours, et que le milieu de culture contient une source de carbone et d'azote assimilables.

**3.** Procédé conforme aux revendications 1 et 2, caractérisé en ce que l'on utilise des cellules en phase de multiplication.

**4.** Procédé conforme aux revendications 1 et 2, caractérisé en ce qu'on utilise des cellules qui ne sont pas en phase de multiplication ("dormantes").

**5.** Procédé conforme aux revendications 1 à 4, caractérisé en ce que la concentration d'acide ∝ cétonique ne dépasse pas 30 g/l.

**6.** Procédé conforme aux revendications 1 à 5, caractérisé en ce que la concentration en biotine est supérieure à 50 micro-grammes/l.

**7.** Procédé conforme aux revendications 1 à 6, caractérisé en ce que l'acide a cétonique utilisé est l'acide ∝ cétoisocaproïque, l'acide ∝-cétoisovalérianique ou bien l'acide ∝ céto-$\beta$-méthyl valérianique.

**8.** Procédé conforme à la revendication 1, caractérisé en ce que l'on utilise corynebacterium glutamicum ATCC 13 032.

**9.** Procédé conforme à la revendication 1, caractérisé en ce que l'on utilise brevibacterium flavum ATCC 14 067.

Revendications pour les États Contractants: DE, FR, GB

**1.** Procédé permettant d'obtenir par fermentation des acides aminés ramifiés aliphatiques à partir de leurs homologues acides ∝ cétoniques, dans un milieu de culture, caractérisé en ce que l'on fait agir une souche de corynebacterium glutumicum ou de brevibacterium flavum sur les acides ∝ cétoniques, et qu'à l'issue de la transformation, on isole les acides aminés formés.

**2.** Procédé conforme à la revendication 1, caractérisé en ce que l'on travaille à un pH compris entre 5 et 9, à une température comprise entre 20 et 45° C, en conditions aérobies pendant une durée de 1 à 10 jours, et que le milieu de culture contient une source de carbone et d'azote assimilables.

**3.** Procédé conforme aux revendications 1 et 2, caractérisé en ce que l'on utilise des cellules en phase de multiplication.

**4.** Procédé conforme aux revendications 1 et 2, caractérisé en ce qu'on utilise des cellules qui ne sont pas en phase de multiplication ("dormantes").

**5.** Procédé conforme aux revendications 1 à 4, caractérisé en ce que la concentration d'acide ∝ cétonique ne dépasse pas 30 g/l.

**6.** Procédé conforme aux revendications 1 à 5, caractérisé en ce que la concentration en biotine est supérieure à 50 micro-grammes/l.

**7.** Procédé conforme aux revendications 1 à 6, caractérisé en ce que l'acide ∝ cétonique utili-

sé est l'acide ∝ cétoisocaproïque, l'acide ∝-cétoisovalérianique ou bien l'acide a céto-$\beta$-méthyl valérianique.

8. Procédé conforme à la revendication 1, caractérisé en ce que l'on utilise corynebacterium glutamicum ATCC 13 032.

9. Procédé conforme à la revendication 1, caractérisé en ce que l'on utilise brevibacterium flavum ATCC 14 067.